# EUROPEAN PATENT APPLICATION

(11) **EP 4 691 460 A1**
(43) Date of publication of application: **11.02.2026**
(21) Application number: 23931687.0
(22) Date of filing: 07.07.2023
(51) Int. Cl.: A61K 9/70, A61K 31/343, A61K 47/38, A61K 47/32, A61P 25/00

(54) **RAMELTEON SUBLINGUAL FILM AND PREPARATION METHOD THEREFOR**

(30) Priority: 03.04.2023 CN 202310343065
(71) Applicant: Hangzhou Chengbang Pharmaceutical Technology Co., Ltd, Hangzhou, Zhejiang 310000 (CN)
(72) Inventor: GE, Zhen, Hangzhou, Zhejiang 310000 (CN); MIAO, Kongsong, Hangzhou, Zhejiang 310000 (CN); YANG, Jun, Hangzhou, Zhejiang 310000 (CN); ZHONG, Shichun, Hangzhou, Zhejiang 310000 (CN)
(74) Representative: Winter, Brandl - Partnerschaft mbB
(86) International application number: PCT/CN2023/106268
(87) International publication number: WO 2024/207637

(57) **Abstract**

A ramelteon sublingual film and a preparation method therefor. The sublingual film at least comprises ramelteon, a film forming material, and one or more of a plasticizer, a disintegrating agent, a flavoring agent or a sweetener, and a stabilizer, wherein the ramelteon is in an amorphous state. The ramelteon is developed into a sublingual film, such that the ramelteon can be administrated through sublingual mucosa. The ramelteon is present in an amorphous state and is thus easier to absorb; the particle size of the ramelteon in the film is molecular-level dispersion, such that the ramelteon can be absorbed better, drugs are not easy to gather and recrystallize, the stability is better, and the solubility is good; the ramelteon can be completely dissolved in water within 1 min and rapidly absorbed by oral mucosa, thereby achieving the objective of rapid onset of action; absolute bioavailability is greater than 30%, and the ramelteon sublingual film has obviously higher bioavailability than that of an original tablet and a common film, and has more rapid onset of action, no food effect, low costs, and less side effects.

## Description

### TECHNICAL FIELD

The disclosure relates to the technical field of pharmaceutical preparations, and in particular relates to a ramelteon sublingual film and preparation method therefor.

### BACKGROUND ART

Ramelteon is developed by Takeda Pharmaceuticals of Japan and was approved by U.S.FDA as an oral hypnotic drug in July 2005. It is the first melatonin receptor agonist used in the clinical treatment of insomnia. It is mainly used to treat insomnia caused by difficulty falling asleep, and also has definite therapeutic effects on chronic insomnia and short-term insomnia.

Ramelteon is a melatonin receptor agonist with high affinity for melatonin Melatonin Type 1 (MT1) and Melatonin Type 2 (MT2) receptors. It acts as a specific full agonist on Melatonin Type 1 and Melatonin Type 2 receptors, but does not act on Melatonin Type 3 receptors. In addition, it does not bind to neurotransmitter receptors such as the GABA receptor complex, and does not interfere with the activity of most enzymes within a certain range. Therefore, it can avoid the distraction, drug addiction and dependence associated with GABA drugs. The total amount of its main metabolite M-II is 20-100 times that of the metabolic substrate, but its activity is lower. The affinity with MT1 and MT2 receptors is about one fifth and one tenth of that of the metabolic substrate, respectively. Compared with the prototype drug, its pharmacological activity is reduced by about 17-25 times. Other metabolites are inactive.

Currently, the marketed product of ramelteon is tablets. Clinical data show that ramelteon is rapidly absorbed after oral administration, with the median time to peak drug concentration being approximately 0.75 (0.5-1.5) h. At least 84% of the drug can be absorbed after oral administration, but its absolute bioavailability is only 1.4% due to the first pass effect. In addition, when taken with a high-fat meal, the AUC is 31% higher than that of fasting administration, the Cmax is reduced by 22%, and the median Tmax is delayed by 45 minutes. Therefore, the key to developing new ramelteon preparations is to develop a new route of administration, select a suitable dosage form, avoid the first pass effect, improve the bioavailability, eliminate the food effect, and shorten the time to peak drug concentration.

CN110996938A discloses a ramelteon composition that is administered via a mucosal delivery system (including intranasal or sublingual) to avoid the first pass effect in the liver. Sulfobutyl ether-β-cyclodextrin is added to the formula to form an inclusion complex to improve the solubility of ramelteon. The process uses wet granulation with ethanol as the solvent. The process steps are cumbersome. The use of organic solvents pollutes the environment and has the risk of explosion. The prepared granules are hard, have a noticeable gritty feeling in the mouth, and have poor patient compliance.

CN103429223A discloses a ramelteon oral mucosal absorption preparation, including sublingual administration or administration by melting in the mouth. The formulation includes a large amount of insoluble excipients including microcrystalline cellulose and pregelatinized starch. The preparation has a noticeable gritty feeling during administration and lacks a taste masking agent. Patient compliance is poor. The preparation process uses a fluidized bed, which is cumbersome and energy-intensive. The median of the time to peak drug concentration is approximately 0.6 (0.5-0.8) h, and the onset of action is still relatively slow.

CN112190555A discloses a ramelteon sublingual tablet and a preparation method thereof. Ramelteon and excipients need to be sieved, which easily generates dust. Excessive inhalation by experimenters can easily cause drowsiness and create production safety issues. The grinding process of ramelteon and mannitol with a ball mill is cumbersome and unsuitable for industrial production. Sublingual tablets are thicker than sublingual films, dissolve more slowly, and are easily lost with saliva.

In summary, the disadvantages of ramelteon preparations in the existing technology are: (1) a noticeable gritty feeling after administration and poor compliance; (2) complex process, poor safety, long production cycle, and high energy consumption; (3) severe loss after disintegration, inaccurate dosage, and large individual differences; (4) the raw material is in a crystalline state and has low solubility in saliva.

### SUMMARY

In order to solve the above technical problems, the present disclosure provides a ramelteon sublingual film and a preparation method therefor.

The technical solutions disclosed herein are specifically as follows.

A sublingual film includes at least ramelteon, a film forming material, and one or more of a plasticizer, a disintegrating agent, a flavoring agent or a sweetener, and a stabilizer, wherein the ramelteon is in an amorphous state.

In the sublingual film, a particle size of the ramelteon in the film is molecular-level dispersion.

In the sublingual film, a weight percentage of the ramelteon is 0.1-5%.

In the sublingual film, a thickness of the film is 5-1000 µm, preferably 10-500 µm, and a weight of the film is 10-400 mg, preferably 10-100 mg.

The sublingual film, according to parts by weight, includes at least 0.1-5 parts by weight of the ramelteon, 10-99 parts by weight of the film forming material, 0.1-20 parts by weight of the plasticizer, 0.1-20 parts by weight of the disintegrating agent, 0.1-10 parts by weight of the flavoring agent or the sweetener, and 0.0-10 parts by weight of the stabilizer.

In the sublingual film, the film forming material is selected from one or more of copovidone, soluplus, povidone, hydroxypropyl methylcellulose, polyvinyl alcohol, polyoxyethylene, polyethylene glycol, hydroxypropyl cellulose, and hydroxyethyl cellulose; preferably, the film forming material is selected from at least one of copovidone, polyoxyethylene, and soluplus; more preferably, the film forming material is selected from copovidone and polyoxyethylene.

In the sublingual film, a weight ratio of the copovidone to the polyoxyethylene is 1: (0.25-4); preferably, the weight ratio of the copovidone to the polyoxyethylene is 1: (0.5-2).

In the sublingual film, the plasticizer is selected from one or more of polyethylene glycol, glycerol, propylene glycol, triacetin, triethyl citrate, sorbitol, mannitol, and dibutyl phthalate.

In the sublingual film, the disintegrating agent is selected from one or more of low-substituted hydroxypropyl methylcellulose, low-substituted hydroxypropyl cellulose, starch, methylcellulose, crospovidone, croscarmellose sodium, polacrilin potassium, and microcrystalline cellulose.

In the sublingual film, the sweetener is selected from one or more of aspartame, mannitol, glycerol, fructose, xylitol, sucralose, sorbitol, saccharin, saccharin sodium, stevioside, sucrose, acesulfame potassium, maltitol, sodium cyclamate, alitame, and lactitol.

In the sublingual film, the flavoring agent is selected from one or more of menthol, sodium citrate, trehalose, anhydrous citric acid, essence, ethyl propionate, and diethyl malonate.

In the sublingual film, the stabilizer is selected from one or more of butylated hydroxyanisole, citric acid, butylated hydroxytoluene, sodium ascorbate, vitamins, sodium sulfite, and fumaric acid.

A preparation method for the sublingual film includes at least the following steps:
(1) weighting the ramelteon, the film forming material, the plasticizer, the disintegrating agent, the flavoring agent or the sweetener, and the stabilizer and adding to a mixer for mixing to obtain a mixture;
(2) adding the mixture into a hot-melt extrusion device at a rate of 0.01-100 kg/h, and stretching the mixture into a film using a film haul-off machine after extrusion, adjusting a rotation speed of the film haul-off machine to obtain the film with different thicknesses and widths;
(3) cutting the prepared film into films with different sizes and shapes using a film cutting machine.

The beneficial effects of the disclosure are:
The ramelteon is developed into a sublingual film, such that the ramelteon can be administrated through sublingual mucosa. The ramelteon is present in an amorphous state and is thus easier to absorb. The particle size of the ramelteon in the film is molecular-level dispersion, such that the ramelteon can be absorbed better. Drugs are not easy to gather and recrystallize, the stability is better, and the solubility is good. The ramelteon can be completely dissolved in water within 1 min and rapidly absorbed by oral mucosa, thereby achieving the objective of rapid onset of action. Absolute bioavailability is greater than 30%. The ramelteon sublingual film has obviously higher bioavailability than that of an original tablet and a common film, and has more rapid onset of action, no food effect, low costs, and less side effects.

The pharmaceutical composition of the present disclosure is absorbed through the oral mucosa and enters the blood by attaching the pharmaceutical preparation to the sublingual of the subject. The absorption is rapid, and the time to peak blood drug concentration (Tmax of about 7 minutes) is greatly shortened compared with the innovator's tablet formulation (Tmax of about 45 minutes). Similar to an injection, the pharmaceutical preparation of the present disclosure is not affected by taking food, has no gritty feeling, and does not require drinking water, which greatly improves patient compliance.

The present disclosure adopts a new film-making process, which can be produced continuously, has a short production cycle, high output, low energy consumption, small production space, low cost, and does not use organic solvents, which is more environmentally friendly and safe. Compared with solvent-based film preparations that cannot form a molecular state distribution, the amorphous film preparation of the disclosure has a faster solubility rate, faster sublingual mucosal absorption, faster onset of effect, better stability, and is not easy to crystallize.

### DETAILED DESCRIPTION

### Comparative Example 1

The prescription is shown in the table below. The specification of ramelteon was 0.8 mg, the single dose was 1 ml, and the batch size was 1000 bottles. The dosage (unit: g) and weight ratio of each component were as follows:

| Components | Ratio (w/v) | Batch prescription quantity |
|---|---|---|
| Ramelteon | 0.08% | 0.8g |
| Sodium chloride (injection grade) | 0.9% | 9g |
| Water (injection grade) added to | 1ml | 1000ml |

Preparation method: taking about 900 ml of water for injection, adding sodium chloride, stirring to dissolve, then adding ramelteon and stirring until completely dissolved, diluting to 1000 ml with water for injection, and dividing it into 1000 bottles, 1 ml per bottle

### Comparative Example 2

A blank film was prepared as a crystal comparison sample, the amount of each component used (unit: g):

| Components | Blank film preparation Batch prescription quantity (g) |
|---|---|
| Copovidone | 8.95 |
| Polyoxyethylene | 8.95 |
| Crospovidone | 0.50 |
| Sorbitol | 0.40 |
| Butylated hydroxytoluene | 0.20 |
| Sucralose | 0.20 |
| Total | 19.20 |

### Process:

According to the prescription, copovidone, polyoxyethylene, crospovidone, sucralose, sorbitol and butylated hydroxytoluene were added to the mixer at 12 rpm and mixed for 5 min. Then the mixture was added to the feeder of the hot-melt extruder, the barrel temperature was set to 80°C, 100°C, 130°C, 140°C, 140°C, 140°C, 140°C, 140°C, the die temperature was 140°C, the haul-off speed was 2, and the screw speed was 50 rpm. After film formation, the film was cut into appropriate size and shape and packaged.

### Example 1

The prescription is shown in the table below. The specification of ramelteon was 2 mg, the weight of a single tablet was 40 mg , and the batch size was 1000 films. The dosage (unit: g) and weight ratio of each component are as follows:

| Components | Proportion (%) | Batch prescription quantity (g) |
|---|---|---|
| Ramelteon | 5.0 | 2.0 |
| Soluplus | 36.0 | 14.4 |
| Polyoxyethylene | 50.0 | 20.0 |
| Microcrystalline cellulose | 2.5 | 1.0 |
| Glycerin | 5.0 | 2.0 |
| Saccharin sodium | 1.5 | 0.6 |
| Total | 100.0 | 40.0 |

### Process:

According to the prescription, ramelteon, soluplus, polyoxyethylene, microcrystalline cellulose and saccharin sodium were added to the mixer at 12 rpm and mixed for 5 min. Then the mixture was added to the feeder of the hot-melt extruder, and glycerol was slowly added to the barrel using a peristaltic pump. The barrel temperature was set to 80°C, 100°C, 120°C, 130°C, 130°C, 130°C, 130°C, 130°C, the die temperature was 130°C, the haul-off speed was 3, and the screw speed was 20 rpm. After film formation, the film was cut into appropriate size and shape and packaged.

### Result:

The ramelteon oral soluble film prepared according to the above prescription and process has good film-forming properties, a smooth surface, uniform color, fast disintegration speed and good mechanical properties. The drug exists in an amorphous state.

Disintegration time: determined by the disintegration time test method in Appendix 0921 of the 2020 edition of the "Chinese Pharmacopoeia".

| Serial number | Thickness /mm | Disintegration time /s |
|---|---|---|
| 1 | 0.07 | 20 |
| 2 | 0.07 | 21 |
| 3 | 0.07 | 20 |
| 4 | 0.07 | 19 |
| 5 | 0.07 | 22 |
| 6 | 0.07 | 23 |

### Tensile test data

| Serial number | Thickness /mm | Tensile strength /MPa |
|---|---|---|
| 1 | 0.07 | 20.15 |
| 2 | 0.07 | 21.87 |
| 3 | 0.07 | 20.56 |

### Example 2

The prescription is shown in the table below. The specification of ramelteon was 2 mg, the weight of a single tablet was 40 mg, and the batch size was 1000 films. The dosage (unit: g) and weight ratio of each component are as follows:

| Components | Proportion (%) | Batch prescription quantity (g) |
|---|---|---|
| Ramelteon | 5.0 | 2.0 |
| Hydroxypropyl methylcellulose | 85.5 | 34.2 |
| Crospovidone | 2.5 | 1.0 |
| Propylene glycol | 5.0 | 2.0 |
| Aspartame | 2.0 | 0.8 |
| Total | 100.0 | 40.0 |

### Process:

According to the prescription, ramelteon, hypromellose and aspartame were added to the mixer at 12 rpm and mixed for 5 min. The mixture was then added to the feeder of the hot-melt extruder, and propylene glycol was slowly added to the barrel using a peristaltic pump. The barrel temperature was set to 100°C, 150°C, 170°C, 170°C, 170°C, 170°C, 170°C, and 170°C, the die temperature was 170°C, the haul-off speed was 2, and the screw speed was 20 rpm. After film formation, the film was cut into appropriate size and shape and packaged.

### Result:

The ramelteon film prepared according to the above prescription and process has good film-forming properties, a smooth surface, uniform color, fast disintegration speed, good mechanical properties. The drug exists in an amorphous state.

Disintegration time: determined by the disintegration time test method in Appendix 0921 of the 2020 edition of the "Chinese Pharmacopoeia".

| Serial number | Thickness /mm | Disintegration time /s |
|---|---|---|
| 1 | 0.05 | 30 |
| 2 | 0.05 | 35 |
| 3 | 0.05 | 38 |
| 4 | 0.05 | 33 |
| 5 | 0.05 | 34 |
| 6 | 0.05 | 35 |

### Tensile test data

| Serial number | Thickness /mm | Tensile strength /MPa |
|---|---|---|
| 1 | 0.05 | 31.46 |
| 2 | 0.05 | 35.70 |
| 3 | 0.05 | 32.91 |

### Example 3

The prescription is shown in the table below. The specification of ramelteon was 2 mg, the weight of a single tablet was 40 mg , and the batch size was 1000 films. The dosage (unit: g) and weight ratio of each component are as follows:

| Components | Proportion (%) | Batch prescription quantity (g) |
|---|---|---|
| Ramelteon | 5 | 2.0 |
| Soluplus | 89 | 35.6 |
| Sorbitol | 3 | 1.2 |
| Croscarmellose sodium | 2 | 0.8 |
| Sucralose | 1 | 0.4 |
| Total | 100 | 40.0 |

### Process:

According to the prescription, ramelteon, soluplus, sorbitol, croscarmellose sodium and sucralose were added to the mixer at 12 rpm and mixed for 5 min. Then the mixture was added to the feeder of the hot-melt extruder, and the barrel temperature was set to 50°C, 70°C, 90°C, 100°C, 100°C, 100°C, 100°C, 100°C, the die temperature was 100°C, the haul-off speed was 1.5, and the screw speed was 20 rpm. After film formation, the film was cut into appropriate size and shape and packaged.

### Result:

The ramelteon film prepared according to the above prescription and process has good film-forming properties and a fast disintegration rate, but has particles on the surface. The drug exists in the form of crystals.

Disintegration time: determined by the disintegration time test method in Appendix 0921 of the 2020 edition of the "Chinese Pharmacopoeia".

### Melting curve

| Time /min | 5 | 10 | 15 | 30 | 45 | 60 |
|---|---|---|---|---|---|---|
| Water | 55 | 73 | 88 | 97 | 99 | 99 |
| pH6.8 | 60 | 75 | 91 | 99 | 100 | 100 |

| Serial number | Thickness /mm | Disintegration time /s |
|---|---|---|
| 1 | 0.07 | 32 |
| 2 | 0.07 | 34 |
| 3 | 0.07 | 31 |
| 4 | 0.07 | 33 |
| 5 | 0.07 | 32 |
| 6 | 0.07 | 32 |

### Tensile test data

| Serial number | Thickness /mm | Tensile strength /MPa |
|---|---|---|
| 1 | 0.07 | 18.97 |
| 2 | 0.07 | 20.11 |
| 3 | 0.07 | 18.77 |

### Example 4

The films of different thicknesses were prepared and the differences in mechanical properties, dissolution and disintegration time were compared. The batch size was 1000 films. The dosage of each component (unit: g) is as follows:

| Components | Proportion (%) | Batch prescription quantity (g) |
|---|---|---|
| Ramelteon | 4.0% | 0.80 |
| Copovidone | 44.75% | 8.95 |
| Polyoxyethylene | 44.75% | 8.95 |
| Crospovidone | 2.5% | 0.50 |
| Sorbitol | 2.0% | 0.40 |
| Butylated hydroxytoluene | 1.0% | 0.20 |
| Sucralose | 1.0% | 0.20 |
| Total | 100% | 20.0 |

### Process:

According to the prescription, ramelteon, copovidone, polyoxyethylene, crospovidone, sucralose, sorbitol and butylated hydroxytoluene were added to a mixer at 12 rpm and mixed for 5 min. Then the mixture was added to the feeder of the hot-melt extruder, the barrel temperature was set to 80°C, 100°C, 130°C, 140°C, 140°C, 140°C, 140°C, 140°C, 140°C, the die temperature was 140°C, the screw speed was 50 rpm, and the haul-off speed was adjusted within the range of 1-5 to obtain film with different thicknesses. After film formation, the film was cut into appropriate size and shape and packaged.

### Result:

The ramelteon film of various thicknesses prepared according to the above prescription and process has good film-forming properties, smooth surface and uniform color.

### Melting curve

| Thickness (µm) | Time /min | 5 | 10 | 15 | 30 | 45 | 60 |
|---|---|---|---|---|---|---|---|
| 10 | pH6.8 | 98 | 100 | 100 | 101 | 100 | 100 |
| 50 | | 95 | 99 | 100 | 100 | 100 | 100 |
| 100 | | 88 | 94 | 101 | 101 | 101 | 101 |
| 500 | | 73 | 86 | 91 | 97 | 100 | 100 |

Disintegration time: Determined by the disintegration time test method in Appendix 0921 of the 2020 edition of the "Chinese Pharmacopoeia".

| Serial number | 10µm | 50µm | 100µm | 500µm |
|---|---|---|---|---|
| | Disintegration time /s | | | |
| 1 | 3 | 8 | 15 | 238 |
| 2 | 2 | 8 | 16 | 240 |
| 3 | 3 | 7 | 13 | 229 |
| 4 | 3 | 8 | 14 | 234 |
| 5 | 4 | 6 | 15 | 241 |
| 6 | 2 | 9 | 15 | 239 |

### Tensile test data

| Serial numbe r | 10µm | | 50µm | | 100µm | | 500µm | |
|---|---|---|---|---|---|---|---|---|
| | Thicknes s/mm | Tensile strengt h /MPa | Thicknes s /mm | Tensile strengt h /MPa | Thicknes s /mm | Tensile strengt h /MPa | Thicknes s /mm | Tensile strengt h /MPa |
| 1 | 0.01 | 2.66 | 0.05 | 11.97 | 0.10 | 19.10 | 0.50 | 30.73 |
| 2 | 0.01 | 1.73 | 0.05 | 12.22 | 0.10 | 19.52 | 0.50 | 38.41 |
| 3 | 0.01 | 2.82 | 0.05 | 11.63 | 0.10 | 18.64 | 0.50 | 34.19 |

Films with thicknesses ranging from 10-500 µm could be formed with good film-forming properties, but the 10 µm film was already relatively soft and had relatively weak mechanical properties. If the thickness was further reduced, it would affect the transportation and administration of the film. Due to the large thickness of 500 µm, the disintegration time was close to 5 min (Chinese Pharmacopoeia 2020 edition).

### Example 5

Films of different specifications were prepared, the effects of different proportions of raw materials on film properties were compared, and the absorption in animals was evaluated. The percentage of each component (unit: % ) is shown in the table below.

Prescription 1: Specification 0.1mg, film weight 100mg, raw material content 0.1%, batch size 1000 films.

Prescription 2: Specification 0.3mg, film weight 30mg, raw material content 1.0%, batch size 1000 films.

Prescription 3: Specification 0.8mg, film weight 20mg, raw material content 4.0%, batch size 1000 films.

Prescription 4: Specification 2.0mg, film weight 40mg, raw material content 5.0%, batch size 1000 films.

Prescription 5: Specification 8.0mg, film weight 80mg, raw material content 10.0%, batch size 1000 films.

| Components | Prescription 1 | Prescription 2 | Prescription 3 | Prescription 4 | Prescription 5 |
|---|---|---|---|---|---|
| | Proportion | Proportion | Proportion | Proportion | Proportion |
| | (%) | (%) | (%) | (%) | (%) |
| Ramelteon | 0.1 | 1.0 | 4.0 | 5.0 | 10.0 |
| Copovidone | 46.7 | 46.25 | 44.75 | 44.25 | 41.75 |
| Polyoxyethyl ene | 46.7 | 46.25 | 44.75 | 44.25 | 41.75 |
| Crospovidone | 2.5 | 2.5 | 2.5 | 2.5 | 2.5 |
| Sorbitol | 2.0 | 2.0 | 2.0 | 2.0 | 2.0 |
| Butylated | | | | | |
| hydroxytolue ne | 1.0 | 1.0 | 1.0 | 1.0 | 1.0 |
| Sucralose | 1.0 | 1.0 | 1.0 | 1.0 | 1.0 |
| Total | 100.0 | 100.0 | 100.0 | 100.0 | 100.0 |

### Process:

According to the prescription, ramelteon, copovidone, polyoxyethylene, crospovidone, sucralose, sorbitol and butylated hydroxytoluene were added to the mixer at 12 rpm and mixed for 5 min. Then the mixture was added to the feeder of the hot-melt extruder, the barrel temperature was set to 80°C, 100°C, 130°C, 140°C, 140°C, 140°C, 140°C, 140°C, the die temperature was 140°C, the haul-off speed was 2.5, and the screw speed was 50 rpm. After film formation, the film was cut into appropriate size and shape and packaged.

### Result:

The ramelteon film prepared according to the above prescription and process has good film-forming properties, a smooth surface, uniform color, and a fast disintegration speed.

### Melting curve

| Prescriptio n | Time /min | 5 | 10 | 15 | 30 | 45 | 60 |
|---|---|---|---|---|---|---|---|
| Prescriptio n 1 | | 95 | 100 | 100 | 101 | 100 | 100 |
| Prescriptio n 2 | | 95 | 99 | 100 | 100 | 100 | 100 |
| Prescriptio n 3 | pH6.8 | 94 | 99 | 101 | 101 | 101 | 101 |
| Prescriptio n 4 | | 90 | 98 | 100 | 100 | 100 | 100 |
| Prescriptio n 5 | | 87 | 98 | 102 | 102 | 102 | 102 |

Disintegration time: determined by the disintegration time test method in Appendix 0921 of the 2020 edition of the "Chinese Pharmacopoeia".

| Serial number | Prescription 1 | Prescription 2 | Prescription 3 | Prescription 4 | Prescription 5 |
|---|---|---|---|---|---|
| | Disintegration time /s | | | | |
| 1 | 14 | 9 | 12 | 20 | 26 |
| 2 | 16 | 8 | 13 | 20 | 25 |
| 3 | 14 | 8 | 15 | 21 | 26 |
| 4 | 15 | 7 | 13 | 23 | 25 |
| 5 | 15 | 9 | 15 | 20 | 25 |
| 6 | 15 | 8 | 12 | 19 | 29 |

### Tensile test data

| Seri al nu mbe r | Prescription 1 | | Prescription 2 | | Prescription 3 | | Prescription 4 | | Prescription 5 | |
|---|---|---|---|---|---|---|---|---|---|---|
| | Thick ness /mm | Tensile strengt h /MPa | Thick ness /mm | Tensile strengt h /MPa | Thic kness /mm | Tensile strengt h /MPa | Thick ness /mm | Tensile strengt h /MPa | Thickn ess /mm | Tensil e strengt h /MPa |
| 1 | 0.06 | 10.55 | 0.06 | 12.33 | 0.1 | 18.76 | 0.12 | 21.43 | 0.12 | 22.67 |
| 2 | 0.06 | 11.21 | 0.06 | 12.19 | 0.1 | 19.52 | 0.12 | 23.81 | 0.13 | 24.12 |
| 3 | 0.06 | 10.98 | 0.06 | 13.72 | 0.1 | 18.77 | 0.12 | 22.79 | 0.12 | 21.97 |

Stability test: after packaging, related substances were tested under 60°C/RH75% for 1 week and 2 weeks respectively. The total impurity results are shown in the table below:

| Experimental conditions | 60 °C/RH75% | | | | |
|---|---|---|---|---|---|
| Time | Prescription 1 | Prescription 2 | Prescription 3 | Prescription 4 | Prescription 5 |
| 0 days | 0.19% | 0.19% | 0.18% | 0.18% | 0.19% |
| 1 Week | 0.25% | 0.24% | 0.24% | 0.23% | 0.23% |
| 2 Week | 0.37% | 0.37% | 0.36% | 0.37% | 0.35% |

The results of Example 5 showed that the disintegration time of formulations with different drug loadings was not much different (the difference was mainly caused by thickness), and all of them disintegrated and dissolved quickly. The results of the rapid stability test showed that the stability of the five formulations was good, and no obvious degradation occured.

### Example 6

Films with different film forming material ratios were prepared and the effects of different film forming material ratios on film properties were compared. The batch size was 1000 films. The dosage of each component (unit: g) was as follows:

Prescription 6: Specification 0.8mg, film weight 20mg, raw material content 4.0%, batch size 1000 films

Prescription 7: Specification 0.4mg, film weight 10mg, raw material content 4.0%, batch size 1000 films

| Components | Prescription 6 | Prescription 3 | Prescription 7 |
|---|---|---|---|
| | Proportion (%) | Proportion (%) | Proportion (%) |
| Ramelteon | 4.0 | 4.0 | 4.0 |
| Copovidone | 17.9 | 44.75 | 71.6 |
| Polyoxyethylene | 71.6 | 44.75 | 17.9 |
| Crospovidone | 2.5 | 2.5 | 2.5 |
| Sorbitol | 2.0 | 2.0 | 2.0 |
| Butylated hydroxytoluene | 1.0 | 1.0 | 1.0 |
| Sucralose | 1.0 | 1.0 | 1.0 |
| Total | 100.0 | 100.0 | 100.0 |

### Process:

According to the prescription, ramelteon, copovidone, polyoxyethylene, crospovidone, sucralose, sorbitol and butylated hydroxytoluene were added to the mixer at 12 rpm and mixed for 5 min. Then the mixture was added to the feeder of the hot-melt extruder, the barrel temperature was set to 80°C, 100°C, 130°C, 140°C, 140°C, 140°C, 140°C, 140°C, the die temperature was 140°C, the haul-off speed was 2, and the screw speed was 50 rpm. After film formation, the film was cut into appropriate size and shape and packaged.

### Result:

The ramelteon film prepared according to the above prescription and process has good film-forming properties, a smooth surface, uniform color, and a fast disintegration speed.

Disintegration time: determined by the disintegration time test method in Appendix 0921 of the 2020 edition of the "Chinese Pharmacopoeia".

| Serial number | Prescription 6 | Prescription 3 | Prescription 7 |
|---|---|---|---|
| | Disintegration time /s | | |
| 1 | 50 | 9 | 8 |
| 2 | 43 | 8 | 9 |
| 3 | 55 | 8 | 9 |
| 4 | 46 | 7 | 11 |
| 5 | 44 | 9 | 10 |
| 6 | 42 | 8 | 9 |

### Tensile test data

| Serial number | Prescription 6 | | Prescription 3 | | Prescription 7 | |
|---|---|---|---|---|---|---|
| | Thickness /mm | Tensile strength /MPa | Thickness /mm | Tensile strength /MPa | Thickness /mm | Tensile strength /MPa |
| 1 | 0.20 | 14.53 | 0.06 | 12.33 | 0.06 | 52.29 |
| 2 | 0.20 | 15.21 | 0.06 | 12.19 | 0.06 | 51.37 |
| 3 | 0.20 | 14.83 | 0.06 | 13.72 | 0.06 | 54.92 |

The mixing ratio of copovidone and polyoxyethylene was within the range of 1: (0.25-4), both of which could form films and the process was feasible. The films with a high copovidone ratio had high mechanical strength and slightly poor ductility. The films with a high polyoxyethylene ratio had low mechanical strength and poor ductility.

### Example 7

Six healthy male Beagle dogs weighing (12±1.0) kg were selected. No medication was used for 14 days before the experiment. The subjects were fasted from 20:00 on the day before the experiment. At 9:00 a.m. on the day of the experiment, the subjects were administered intravenous injection (comparative example 1 sample), oral administration (innovator's formulation ROZEREM, 8 mg specification), and sublingual administration (prescriptions 2, 3, 4, and 5), and the blood drug concentrations were measured . After the determination, eluted for 14 D, and the drug was administered again to determine the blood drug concentration. The Tmax, Cmax and AUC of the drugs administered by different routes were compared. The sampling time were 5min, 10min, 15min, 30min, 1h, 2h, 4h and 6h.

| Route of administration | Preparation | Dosage (mg) | Tmax (min) | Cmax (ng/ml) | AUC (h*ng/ml) | BA (%) |
|---|---|---|---|---|---|---|
| Intravenous injection | Injections | 0.8 | 5 | 84.17 | 44.03 | - |
| Oral | Tablet | 8 | 30 | 14.50 | 8.82 | 2.0% |
| Sublingual | Film (Prescription 5) | 8 | 7 | 405.77 | 171.16 | 38.9% |
| | Film (Prescription 4) | 2 | 7 | 200.19 | 45.29 | 41.1% |
| | Film (Prescription 3) | 0.8 | 7 | 35.21 | 22.91 | 52.0% |
| | Film (Prescription 2) | 0.3 | 7 | 16.33 | 9.51 | 57.6% |
| On the tongue | Film (Prescription 5) | 8 | 15 | 91.02 | 39.41 | 8.9% |

The data from Example 7 showed that oral absorption could increase the bioavailability of ramelteon. Compared with the oral innovator's tablet formulation, the relative bioavailability of the sublingual film was increased by nearly 29 times , and the time to peak drug concentration was shortened to 7 min, which was close to that of the injection. It showed that the performance of the sublingual film in this disclosure was better than the innovator's tablet formulation. The same effect only required a smaller dose and could fall asleep faster.

The above description is only a preferred embodiment of the present disclosure and is not intended to limit the present disclosure. Any modifications, equivalent substitutions and improvements, etc. made within the spirit and principle of the present disclosure are included in the protection scope of the present disclosure.

## Claims

1. A sublingual film, **characterized in that**, the sublingual film comprises at least ramelteon, a film forming material, and one or more of a plasticizer, a disintegrating agent, a flavoring agent or a sweetener, and a stabilizer, wherein the ramelteon is in an amorphous state.

2. The sublingual film according to claim 1, **characterized in that**, a particle size of the ramelteon in the film is molecular-level dispersion.

3. The sublingual film according to claim 1, **characterized in that**, a weight percentage of the ramelteon is 0.1-5%.

4. The sublingual film according to claim 1, **characterized in that**, a thickness of the film is 5-1000 µm, preferably 10-500 µm, and a weight of the film is 10-400 mg, preferably 10-100 mg.

5. The sublingual film according to claim 1, **characterized in that**, according to parts by weight,the sublingual film comprises at least 0.1-5 parts by weight of the ramelteon, 10-99 parts by weight of the film forming material, 0.1-20 parts by weight of the plasticizer, 0.1-20 parts by weight of the disintegrating agent, 0.1-10 parts by weight of the flavoring agent or the sweetener, and 0.0-10 parts by weight of the stabilizer.

6. The sublingual film according to claim 1, **characterized in that**, the film forming material is selected from one or more of copovidone, soluplus, povidone, hydroxypropyl methylcellulose, polyvinyl alcohol, polyoxyethylene, polyethylene glycol, hydroxypropyl cellulose, and hydroxyethyl cellulose; preferably, the film forming material is selected from at least one of copovidone, polyoxyethylene, and soluplus; more preferably, the film forming material is selected from copovidone and polyoxyethylene.

7. The sublingual film according to claim 6, **characterized in that**, a weight ratio of the copovidone to the polyoxyethylene is 1: (0.25-4); preferably, the weight ratio of the copovidone to the polyoxyethylene is 1: (0.5-2).

8. The sublingual film according to claim 1, **characterized in that**, the plasticizer is selected from one or more of polyethylene glycol, glycerol, propylene glycol, triacetin, triethyl citrate, sorbitol, mannitol, and dibutyl phthalate.

9. The sublingual film according to claim 1, **characterized in that**, the disintegrating agent is selected from one or more of low-substituted hydroxypropyl methylcellulose, low-substituted hydroxypropyl cellulose, starch, methylcellulose, crospovidone, croscarmellose sodium, polacrilin potassium, and microcrystalline cellulose.

10. The sublingual film according to claim 1, **characterized in that**, the sweetener is selected from one or more of aspartame, mannitol, glycerol, fructose, xylitol, sucralose, sorbitol, saccharin, saccharin sodium, stevioside, sucrose, acesulfame potassium, maltitol, sodium cyclamate, alitame, and lactitol.

11. The sublingual film according to claim 1, **characterized in that**, the flavoring agent is selected from one or more of menthol, sodium citrate, trehalose, anhydrous citric acid, essence, ethyl propionate, and diethyl malonate.

12. The sublingual film according to claim 1, **characterized in that**, the stabilizer is selected from one or more of butylated hydroxyanisole, citric acid, butylated hydroxytoluene, sodium ascorbate, vitamins, sodium sulfite, and fumaric acid.

13. A preparation method for the sublingual film according to any one of claims 1-12, **characterized in that**, comprising following steps:
(1) weighting the ramelteon, the film forming material, the plasticizer, the disintegrating agent, the flavoring agent or the sweetener, and the stabilizer and adding to a mixer for mixing to obtain a mixture;
(2) adding the mixture into a hot-melt extrusion device at a rate of 0.01-100 kg/h, and stretching the mixture into a film using a film haul-off machine after extrusion, adjusting a rotation speed of the film haul-off machine to obtain the film with different thicknesses and widths;
(3) cutting the prepared film into films with different sizes and shapes using a film cutting machine.
